Europäisches Patentamt

European Patent Office

Office européen des brevets

(11) Veröffentlichungsnummer: **0 301 356 B1**

## (12) EUROPÄISCHE PATENTSCHRIFT

(45) Veröffentlichungstag der Patentschrift: **24.06.92**

(21) Anmeldenummer: **88111537.2**

(22) Anmeldetag: **18.07.88**

(51) Int. Cl.⁵: $H05G\ 1/64$, H05G 1/02,
G03B 42/02

(54) **Lichtverteiler für eine Röntgendiagnostikeinrichtung.**

(30) Priorität: **29.07.87 DE 8710425 U**

(43) Veröffentlichungstag der Anmeldung:
**01.02.89 Patentblatt 89/05**

(45) Bekanntmachung des Hinweises auf die
Patenterteilung:
**24.06.92 Patentblatt 92/26**

(84) Benannte Vertragsstaaten:
**DE FR**

(56) Entgegenhaltungen:
**EP-A- 0 051 430      EP-A- 0 052 995**
**US-A- 3 622 786      US-A- 3 684 354**
**US-A- 4 058 833      US-A- 4 063 092**

(73) Patentinhaber: **SIEMENS AKTIENGESELL-
SCHAFT
Wittelsbacherplatz 2
W-8000 München 2(DE)**

(72) Erfinder: **Weiss, Karl
Im Föhrenwald 23
W-8520 Buckenhof(DE)**
Erfinder: **Gall, Arthur, Dipl.-Ing.
Fichtenstrasse 1
W-8521 Langensendelbach(DE)**

EP 0 301 356 B1

Rank Xerox (UK) Business Services

**Beschreibung**

Die Erfindung betrifft einen Lichtverteiler für eine Röntgendiagnostikeinrichtung mit einem Gehäuse einem ersten und zweiten Spiegel zur wahlweisen Ablenkung des Strahlenganges des Ausgangsbildes eines Röntgenbildverstärkers auf an dem Gehäuse des Lichtverteilers befestigte Aufnahmekomponenten. Derartige Lichtverteiler ermöglichen den seriellen oder parallelen Betrieb von verschiedenen Aufnahmegeräten, wie beispeilsweise eine Fernseh-, eine Kinound eine Blattfilm- oder Keinbildkamera, zur Aufnahme von Ausgangsbilder eines Röntgenbildverstärkers.

In der US-PS-4 383 328 ist ein derartiger Lichtverteiler beschrieben, der das Ausgangsbild des Röntgenbildverstärkers auf zwei Kinokameras und eine Fernsehkamera verteilt. Ein klappbar angebrachter halbdurchlässiger Spiegel bewirkt die Aufteilung auf zwei Kinokameras. Dieser Klappspiegel bleibt aber in seiner Parkstellung innerhalb des Strahlenganges einer Kinokamera. Vor dem klappbaren Spiegel in seiner Ruhestellung läßt sich ein weiterer Spiegel mit einer Reflexion von 100 % einschieben, der senkrecht zum Strahlengang des Röntgenbildverstärkers angeordnet ist. Eine Spiegelanordnung mit einem um 45° gekippten drehbaren Spiegel mit einer Reflexion von 90 % läßt sich im Strahlengang vor den einschiebbaren Spiegel einfahren, der eine wahlweise Ablenkung des Strahlenganges auf eine der Kinokameras oder eine Fernsehkamera ermöglicht. Es wurde zwar bei diesem Lichtverteiler Wert darauf gelegt, einen möglichst kompakten Aufbau zu erreichen. Durch die Anordnung einer der Aufnahmekomponenten, beispielsweise der ersten Kinokamera, in dem direkten Strahlengang des Röntgenbildverstärkers erhöht sich die Baulänge der Röntgenbildverstärker-Aufnahmeeinheit in unerwünschter Weise, so daß in einigen Fällen die Raumhöhe des Untersuchungsraumes bei der Verwendung von Untertischgeräten und einem zusätzlichen Deckenstativ nicht ausreicht.

Die Erfindung geht von der Aufgabe aus, einen Lichtverteiler der eingangs genannten Art zu schaffen, der einen stabilen und kompakten Aufbau aufweist und bei dem die Aufnahmekomponenten derart angeordnet sind, daß sie zur Bauhöhe in Richtung des Röntgenbildverstärkers nicht beitragen.

Die Aufgabe wird erfindungsgemaß dadurch gelöst, daß sämtliche Aufnahmekomponenten seitlich an dem Lichtverteiler angeordnet sind, daß die Achsen des Strahlenganges der Aufnahmekomponenten senkrecht aufeinander und jeweils senkrecht auf der Achse des Strahlenganges des Röntgenbildverstärkers stehen, daß der erste Spiegel den Strahlengang des Röntgenbildverstärkers auf die erste Aufnahmekomponente lenkt und daß der zweite Spiegel in den Strahlengang zwischen erstem Spiegel und erster Aufnahmekomponente einfahrbar ist und den Strahlengang auf die zweite Aufnahmekomponente ablenkt. Durch diese Maßnahmen wird erreicht, daß sämtliche Aufnahmekomponenten seitlich an dem Lichtverteiler angebracht sind, so daß sie in Richtung der Bildverstärkerachse keinen Beitrag zur Bauhöhe bewirken.

Eine Mitbeobachtung, beispielsweise durch eine Fernsehkamera als erste Aufnahmekomponente, bei Betrieb der zweiten Aufnahmekomponente wird erreicht, wenn der zweite Spiegel ein teildurchlässiger Spiegel ist. Eine stabile und leicht zu justierende Anordnung des beweglichen Spiegels erhält man, wenn zwei Führungsstangen vorgesehen sind, die schräg zum Strahlengang zwischen erstem Spiegel und erster Aufnahmekomponente angeordnet sind und diesen umgeben und wenn der zweite Spiegel auf einem Schlitten befestigt ist, der auf der einen Seite Kugelbüchsen aufweist, die die erste Führungsstange umgeben, und über Rollager mit der zweiten Führungsstange verbunden ist. Einen einfachen Antrieb des Schlittens für den beweglichen Spiegel erhält man, wenn der Schlitten mit einem einseitig am Gehäuse befestigten Kurbeltrieb verbunden ist, der mit einem Drehantrieb versehen ist.

Die Erfindung ist nachfolgend anhand eines in der Zeichnung dargestellten Ausführungsbeispieles näher erläutert. Es zeigen:

Fig. 1 eine Röntgendiagnostikeinrichtung mit einem Lichtverteiler nach dem Stand der Technik,

Fig. 2 eine Darstellung des Lichtverteilers von unten aus der Sicht des Röntgenbildverstärkers, und

Fig. 3 eine Draufsicht auf den erfindungsgemäßen Lichtverteiler.

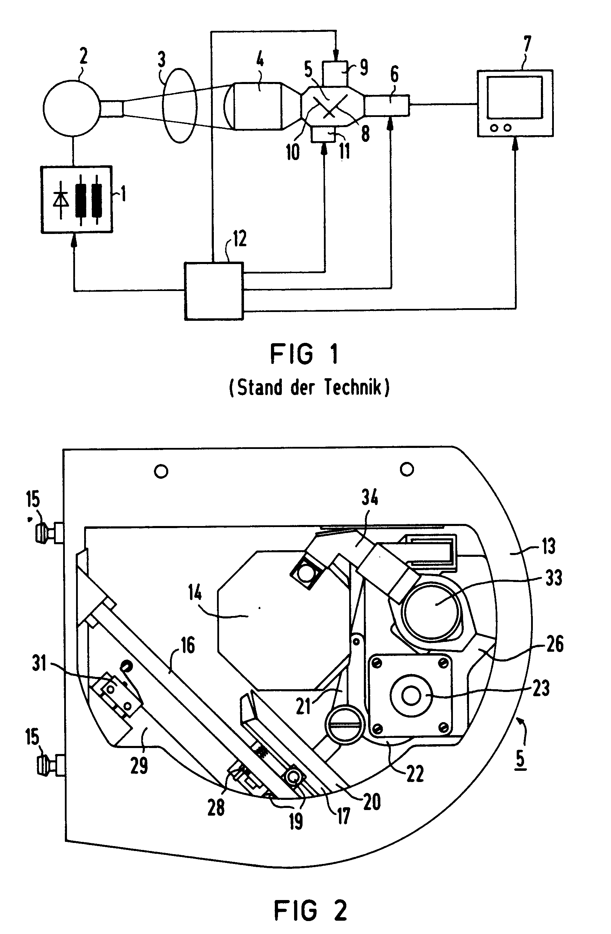

In der Fig. 1 ist eine Röntgendiagnostikeinrichtung nach dem Stand der Technik mit einem Hochspannungsgenerator 1 dargestellt, der eine Röntgenröhre 2 betreibt. Die Röntgenröhre 2 sendet ein Strahlenbündel aus, das einen Patienten 3 durchdringt und auf den Eingangsleuchtschirm eines Röntgenbildverstärkers 4 fällt. An dem Ausgang des Röntgenbildverstärkers 4 ist ein Lichtverteiler 5 angekoppelt, an dessen einem Ausgang eine Fernsehkamera 6 als eine erste Aufnahmekomponente befestigt ist, die das Ausgangsbild des Röntgenbildverstärkers 4 auf einem Monitor 7 darstellt. Durch einen ersten beweglichen, in den Strahlengang des Röntgenbildverstärkers 4 einbringbaren Spiegel 8 wird das Ausgangsbild des Röntgenbildverstärkers 4 auf beispielsweise eine Kinokamera 9 als eine zweite Aufnahmekomponente abgelenkt. Ein zweiter beweglicher, ebenfalls in den Strahlengang einbringbarer Spiegel 10 lenkt den Strahlengang in eine andere Richtung ab, so daß das Ausgangsbild des Röntgenbildverstärkers 4 beispielsweise von einer Fotokamera 11 als eine dritte Aufnahmekomponente aufgenommen werden kann. Eine Zentraleinheit 12 bewirkt dabei die Steuerung und Synchronisation des Hochspannungsgenerators 1, der Fernsehkette 6 und 7, der Spiegel 8 und 10 sowie der Kameras 9 und 11.

Wird eine derartige Röntgendiagnostikeinrichtung als Untertischgerät eingesetzt, bei dem die Röntgenröhre 2 unterhalb des Patientenlagerungstisches und die Bildverstärker-Aufnahmeeinheit 4 bis 11 oberhalb des Patientenlagerungstisches angeordnet ist, ergibt sich die maximal benötigte Raumhöhe aus der Höhe der vollständig hochgefahrenen Röntgenbildverstärker-Aufnahmeeinheit 4 bis 11. Die vom Röntgenbildverstärker 4 wegweisende Fernsehkamera 6 erhöht dabei den maximalen Raumbedarf in unerwünschter Weise.

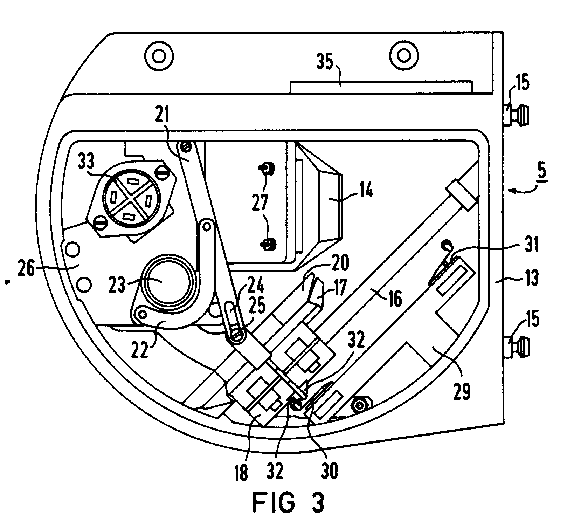

In der Fig. 2 ist ein Gehäuse 13 eines erfindungsgemäßen Lichtverteilers 5 von unten aus der Sicht des Röntgenbildverstärkers 4 dargestellt. In dem Strahlengang des Röntgenbildverstärkers befindet sich innerhalb des Gehäuses 13 ein erster Spiegel 14, der den Strahlengang nahezu rechtwinklig auf eine an dem Gehäuse 13 durch Befestigungspunkte 15 ankuppelbare Fernsehkamera 6 als eine erste Aufnahmekomponente lenkt. Dieser erste Spiegel 14 kann fest mit dem Gehäuse 13 verbunden sein. Den Strahlengang zwischen dem ersten Spiegel 14 und der ankuppelbaren Fernsehkamera umgeben zwei Führungsstangen 16, auf denen ein Schlitten 17 beweglich gehalten ist. Der Schlitten 17 weist, wie aus Fig. 3 ersichtlich, die eine Draufsicht des in Fig. 2 dargestellten Lichtverteilers 5 wiedergibt, Kugelbüchsen 18 auf, die eine der Führungsstangen 16 umgreifen, während die andere Seite mit Rollen 19 versehen ist, die die zweite Führungsstange 16 als Rollager beidseitig umfassen, so daß der Schlitten 17 entlang der Führungsstangen 16 verfahrbar ist. Auf dem Schlitten 17 ist ein zweiter Spiegel 20 als beweglicher Spiegel befestigt. Dieser zweite bewegliche Spiegel 20 läßt sich durch einen Kurventrieb in den Strahlengang zwischen erstem Spiegel 14 und Fernsehkamera 6 verfahren, so daß der Spiegel 20 den Strahlengang auf eine an dem Gehäuse 13 an eine Halterung 35 ankuppelbare fotografische Kamera 9 bzw. 11 als eine zweite Aufnahmekomponente lenkt.

Der Kurbeltrieb umfaßt eine Kurbelschwinge 21, einen bogenförmig gekrümmten Arm 22 und einen Drehantrieb 23, beispielsweise einen Motor. Die Kurbelschwinge 21 ist einseitig mit dem Gehäuse 13 befestigt. Auf der anderen Seite weist die Kurbelschwinge 21 ein Langloch 24 auf, in das ein an dem Schlitten 17 befestigter Bolzen 25 eingreift. Der Drehantrieb 23 ist über einen ersten Träger 26 mit dem Gehäuse 13 verbunden. Die Spiegel 14 und 20 lassen sich durch Justierschrauben 27 und 28 einstellen.

Unter dem Schlitten 17 ist ein zweiter Träger 29 an dem Gehäuse 13 befestigt, der zwei Mikroschalter 30 und 31 trägt, die als Endschalter für den zweiten beweglichen Spiegel 20 dienen. Hierzu ist der Schlitten 17 mit Nasen 32 versehen, die die Mikroschalter 30 und 31 in den Endstellungen des Schlittens 17 betätigen.

An dem ersten Träger 26 ist weiterhin ein Fotomultiplier 33 befestigt, dem über einen Lichtleiter 34 ein kleiner Teil des Lichtes des Strahlenganges des Röntgenbildverstärkers 4 vor dem ersten Spiegel 14 seitlich ausgekoppelt wird, so daß in bekannter Weise die Helligkeit des Ausgangsbildes des Röntgenbildverstärkers 4 und damit die Röntgenröhrenstrahlung geregelt werden können.

Anstelle der festen Anordnung des ersten Spiegels 14 kann dieser entweder dreh- oder schwenkbar mit dem Gehäuse 13 verbunden sein, so daß sich auch noch weitere Aufnahmekomponenten seitlich anschließen lassen.

Durch diese erfindungsgemäße Anordnung erhält man einen äußerst kompakten Lichtverteiler 5, der den Strahlengang des Röntgenbildverstärkers 4 jeweils auf die seitlich angebrachten Aufnahmekomponenten 6, 9 umlenkt, so daß sich nur eine geringe Höhe des Lichtverteilers 5 mit angekoppelten Aufnahmekomponenten 6, 9 ergibt. Gleichzeitig weist er eine stabile Spiegelanordnung auf. Ebenfalls enthält er nur wenige einfache Steuermittel, so daß der Lichtverteiler 5 nur ein geringes Gewicht aufweist und wenig störanfällig ist.

**Patentansprüche**

1. Lichtverteiler (5) für eine Röntgendiagnostikeinrichtung mit einem Gehäuse (13) und einem ersten und zweiten Spiegel (14, 20) zur wahlweisen Ablenkung des Strahlenganges des Ausgangsbildes eines Röntgenbildverstärkers (4) auf an dem Gehäuse (13) des Lichtverteilers (5) befestigte Aufnahmekomponenten (6, 9, 11), **dadurch gekennzeichnet,** daß sämtliche Aufnahmekomponenten (6, 9, 11) seitlich an dem Lichtverteiler (5) angeordnet sind, daß die Achsen des Strahlenganges der Aufnahmekomponenten (6, 9, 11) senkrecht aufeinander und jeweils senkrecht auf der Achse des Strahlenganges des Röntgenbildverstärkers (4) stehen, daß der erste Spiegel (14) den Strahlengang des Röntgenbildverstärkers (4) auf die erste Aufnahmekomponente (6) lenkt und daß der zweite Spiegel (20) in den Strahlengang zwischen erstem Spiegel (14) und erster Aufnahmekomponente (6) linear einfahrbar ist und den Strahlengang auf die zweite Aufnahmekomponente (9, 11) ablenkt.

2. Lichtverteiler nach Anspruch 1, **dadurch gekennzeichnet,** daß der zweite Spiegel (20) ein teildurchlässiger Spiegel ist.

3. Lichtverteiler nach Anspruch 1 oder 2, **dadurch gekennzeichnet,** daß zwei Führungsstangen (16) vorgesehen sind, die schräg zum Strahlengang zwischen erstem Spiegel (14) und erster Aufnahmekomponente (6) angeordnet sind und diesen umgeben und daß der zweite Spiegel (20) auf einem Schlitten (17) befestigt ist, der auf der einen Seite Kugelbüchsen (18) aufweist, die die erste Führungsstange (16) umgeben und über Rollager (19) mit der zweiten Führungsstange (16) verbunden ist.

4. Lichtverteiler nach Anspruch 3, **dadurch gekennzeichnet,** daß der Schlitten (17) mit einem einseitig am Gehäuse (13) befestigten Kurbeltrieb (21 bis 23) verbunden ist, der mit einem Drehantrieb (23) versehen ist.

## Claims

1. Light distributor (5) for an X-ray diagnostics installation having a housing (13) and a first and second mirror (14, 20) for the optional deflection of the beam path of the output image of an X-ray image intensifier (4) onto detector components (6, 9, 11) secured on the housing (13) of the light distributor (5), characterised in that all the detector components (6, 9, 11) are arranged laterally on the light distributor (5), in that the axes of the beam path of the detector components (6, 9, 11) are perpendicular with respect to each other and respectively perpendicular to the axis of the beam path of the X-ray image intensifier (4), in that the first mirror (14) deflects the beam path of the X-ray image intensifier (4) onto the first detector component (6) and in that the second mirror (20) can be introduced linearly into the beam path between the first mirror (14) and the first detector component (6) and deflects the beam path onto the second detector component (9, 11).

2. Light distributor according to claim 1, characterised in that the second mirror (20) is a partially transparent mirror.

3. Light distributor according to claim 1 or 2, characterised in that two guide rods (16) are provided which are arranged obliquely to the beam path between the first mirror (14) and the first detector component (6) and surround it, and in that the second mirror (20) is secured on a carriage (17) which on one side has ball boxes (18) which surround the first guide rod (16) and is connected to the second guide rod (16) by means of roller bearings (19).

4. Light distributor according to claim 3, characterised in that the carriage (17) is connected to a crank assembly (21 to 23) secured on one side of the housing (13), which assembly is provided with a rotary drive (23).

## Revendications

1. Répartiteur de lumière (5) pour une installation de radiodiagnostic comportant un boîtier (13) et un premier miroir (14) et un second miroir (20) pour dévier au choix le trajet de rayonnement de l'image de sortie d'un amplificateur de brillance (4) en direction des composants de réception (6,9,11) fixés sur le boitier (13) du répartiteur de lumière (5), caractérisé par le fait que tous les composants de réception (6,9,11) sont disposés latéralement sur le répartiteur de lumière (5), que les axes du trajet de rayonnement des composants de réception (6,9,11) sont perpendiculaires entre eux et perpendiculaires chacun à l'axe du trajet de rayonnement de l'amplificateur de brillance (4), que le premier miroir (14) dévie le trajet de rayonnement de l'amplificateur de brillance (4) en direction du premier composant de réception (6), et que le second miroir (20) peut être introduit linéairement dans le trajet du rayonnement entre le premier miroir (14) et le premier composant de réception (6) et dévie le trajet du rayonnement en direction du second composant de réception (9,11).

2. Répartiteur de lumière suivant la revendication 1, caractérisé par le fait que le second miroir (20) est un miroir semi-tranparent.

3. Répartiteur de lumière suivant la revendication 1 ou 2, caractérisé par le fait qu'il est prévu deux barres de guidage (16), qui sont disposées obliquement par rapport au trajet du rayonnement entre le premier miroir (14) et le premier composant de réception (6) et entourent ces derniers, et que le second miroir (20) est fixé sur un chariot (17), qui possède, sur un côté, des douilles à billes (18), qui entourent la première barre de guidage (16), et est raccordé par l'intermédiaire de roulements (19) à la seconde barre de guidage (16).

4. Répartiteur de lumière suivant la revendication 3, caractérisé par le fait que le chariot (17) est raccordé à un dispositif d'entraînement à manivelle (21 à 23), qui est fixé sur un côté du boîtier (13) et comporte un dispositif d'entraînement en rotation (23).

FIG 1

(Stand der Technik)

FIG 2

FIG 3